# FASCICULE DE BREVET EUROPEEN

(11) **EP 2 080 028 B1**
(45) Date de publication et mention de la délivrance du brevet: **25.07.2012**
(21) Numéro de dépôt: 07848315.3
(22) Date de dépôt: 03.10.2007
(51) Int. Cl.: C12R 1/35, G01N 33/569, G01N 33/68, C12Q 1/68

(54) **UTILISATION DE LA PROTEINE 7F4 DANS LE DIAGNOSTIC IN VITRO D'INFECTIONS A MYCOPLASMA PNEUMONIAE**
VERWENDUNG DES PROTEINS 7F4 BEI DER IN-VITRO-DIAGNOSE VON INFEKTIONEN MIT MYCOPLASMA PNEUMONIAE
USE OF THE 7F4 PROTEIN IN THE IN VITRO DIAGNOSIS OF MYCOPLASMA PNEUMONIAE INFECTIONS

(30) Priorité: 05.10.2006 FR 0608734
(43) Date de publication de la demande: 22.07.2009
(73) Titulaire: InGen Biosciences, 91380 Chilly Mazarin (FR); Université Victor Segalen Bordeaux 2, 33076 Bordeaux Cedex (FR)
(72) Inventeur: CYNCYNATUS, Camille, F-94410 Saint Maurice (FR); NUYTTENS, Hélène, F-75014 Paris (FR)
(74) Mandataire: Jacobson, Claude
(86) Numéro de dépôt international: PCT/FR2007/001618
(87) Numéro de publication internationale: WO 2008/040883

(56) Documents cités:
- EP-A- 0 475 185
- EP-A- 1 098 001
- WO-A-96/28472
- US-A- 5 788 962
- ZILBERSTEIN D ET AL: "THE BETA-SUBUNIT OF THE F-1F-0 ATPASE IS CONSERVED IN MYCOPLASMAS" JOURNAL OF BIOLOGICAL CHEMISTRY, vol. 261, no. 16, 1986, pages 7109-7111, XP002429180 ISSN: 0021-9258
- SJOSTROM K E ET AL: "DISTINCTIVE ANTIGENIC SPECIFICITIES OF ATPASE AND NADH DEHYDROGENASES AS MEANS FOR CLASSIFICATION OF THE ORDER MYCOPLASMATALES" INTERNATIONAL JOURNAL OF SYSTEMATIC BACTERIOLOGY, vol. 33, no. 2, 1983, pages 218-228, XP008077764 ISSN: 0020-7713
- DATABASE UniProt [Online] 1 novembre 1997 (1997-11-01), "ATP synthase subunit beta (EC 3.6.3.14) (ATPase subunit beta) (ATP synthase F1 sector subunit beta)." XP002429334 extrait de EBI accession no. UNIPROT:Q50331 Database accession no. Q50331
- SHIMIZU TAKASHI ET AL: "A dipalmitoylated lipoprotein from Mycoplasma pneumoniae activates NF-kappa B through TLR1, TLR2, and TLR6." JOURNAL OF IMMUNOLOGY (BALTIMORE, MD. : 1950) 1 OCT 2005, vol. 175, no. 7, 1 octobre 2005 (2005-10-01), pages 4641-4646, XP002429117 ISSN: 0022-1767
- SASAKI T ET AL: "CROSS-REACTIVE ANTIBODIES TO MYCOPLASMAS FOUND IN HUMAN SERA BY THEENZYME-LINKED IMMUNOSORBENT ASSAY (ELISA)" MICROBIOLOGY AND IMMUNOLOGY, TOKYO, JP, vol. 31, no. 6, 1987, pages 521-530, XP000892669 ISSN: 0385-5600

## Description

La présente invention concerne l'utilisation d'un polypeptide et d'un polynucléotide dans le domaine du diagnostic sérologique d'infections à *Mycoplasma pneumoniae.*

*Mycoplasma pneumoniae* est une bactérie à croissance intracellulaire dépourvue de paroi et qui envahit les cellules de l'épithélium respiratoire. Elle est responsable d'infections respiratoires aiguës fréquemment rencontrées chez l'enfant à partir de cinq ans et chez l'adulte jeune. Il peut s'agir de pneumonies atypiques à évolution favorable parfois associées à d'autres manifestations ORL, cutanées, hématologiques, neurologiques ou plus souvent de trachéobronchites. *Mycoplasma pneumoniae* serait responsable de 15 à 20% des pneumopathies communautaires se manifestant à l'état endémique avec de petites poussées épidémiques tous les quatre à sept ans. (Bébéar C, Bébéar C.M et de Barbeyrac B. dans FreneyJ, Renaud F, Hansen W, Bollet C, Précis de bactériologie clinique, ed. ESKA, Paris, 2000).

Il existe un premier pic d'incidence chez les enfants de 5 à 15 ans, chez qui *Mycoplasma pneumoniae* serait responsable de près de 40% des pneumonies communautaires dont près de 20% nécessitent une hospitalisation. Le second pic d'incidence concerne l'adulte après 50 ans, avec des chiffres s'élevant progressivement avec l'âge jusqu'à dépasser 30% (Brunner H, Mycoplasma pneumoniae infections. Isr. J. Med Sci. (1981), 17:516-523; Ghosh et al. Surveillance of Mycoplasma pneumoniae infections in Scotland 1986-1991. J Infect. (1992), 25: 221-227; Waites et al. Mycoplasma pneumoniae and its role as a human pathogen. Clin. Microbiol. Rev. (2004), 17: 697-728). Le délai important entre la primo-infection chez l'enfant et la réinfection chez l'adulte montre que l'infection chez l'enfant confère à l'individu une protection immunitaire efficace contre la réinfection.

Il a également été observé une association clinique entre *M. pneumoniae* et asthme chez les enfants, même si la nature de la corrélation n'est pas encore clairement établie. (Hansbro PM et al., Role of atypical bacterial infection of the lung in predisposition/protection of asthma. Pharmacology et Therapeutics (2004), 101 : 193-210).

Il semblerait qu'il existe une sous-estimation fréquente des infections aiguës, voire chroniques, à *M. pneumoniae* chez les enfants à travers des pathologies telles que l'asthme ou les pneumopathies communautaires.

*Mycoplasma pneumoniae* pénètre dans l'organisme par voie aérienne (inhalation de gouttelettes, contacts directs avec des sujets infectés) en adhérant aux cellules de l'épithélium respiratoire. Ce contact engendre un stress oxydatif à l'origine de l'altération du mouvement ciliaire et la formation de lésions cellulaires. Une réaction inflammatoire locale se forme. La réaction immunologique peut entraîner l'apparition d'infiltrats et parfois même d'auto-anticorps. Certains antigènes membranaires de *M. pneumoniae* sont en effet semblables à des antigènes retrouvés au niveau du cerveau et du pancréas. (Waites KB, Talkington DF. Mycoplasma pneumoniae and its role as a human pathogen, Clin Microbiol Rev, 2004) .

De nombreux agents infectieux, qu'ils soient viraux ou bactériens, peuvent être à l'origine d'une pneumonie et la symptomatologie respiratoire ne permet guère de différencier les infections à *M. pneumoniae* de celles provoquées par d'autres agents de pneumonies atypiques. Le début de la maladie est progressif après une incubation de 15 à 20 jours. La maladie se manifeste par de la fièvre, un malaise, des céphalées, des myalgies et rachialgies et surtout une toux sèche et opiniâtre. L'état général est peu altéré et l'examen physique pauvre en symptômes, contrastant avec l'importance des images radiologiques des poumons. La maladie est généralement régressive avec le temps, cependant la convalescence est longue et la toux persistante. *M. pneumoniae* peut également causer diverses complications extrapulmonaires en se dispersant au niveau d'autres organes : pleurésies, éruptions cutanées, sinusites, myocardites, péricardites, atteintes articulaires, anémie hémolytique, manifestations nerveuses, infections génitales. (Waites KB, Talkington DF. Mycoplasma pneumoniae and its role as a human pathogen, Clin Microbiol Rev, 2004).

Le traitement des infections à *Mycoplasma pneumoniae* repose sur un choix probabiliste en fonction de l'âge, surtout chez les enfants, et de critères cliniques et radiologiques dont aucun n'est ni très spécifique ni sensible.

L'absence de paroi rend cette bactérie insensible à la pénicilline et autres beta-lactamines habituellement prescrites en cas de pneumonie d'origine bactérienne. Ainsi, les antibiotiques fréquemment utilisés dans les infections à *M. pneumoniae* sont les tétracyclines, les fluoroquinolones, les macrolides et apparentés. Cependant des cas de résistance aux fluoroquinolones et macrolides ont déjà pu être observés (Matsuoka, et al. Characterization and Molecular analysis of Macrolide-Resistant Mycoplasma pneumoniae clinical isolates obtained in Japan. Antimicrob Agents Chemother, 2004, 48, 12 : 4624-4630 ; Gruson D. et al. In vitro developpement of résistance to six and four fluoroquinolones in Mycoplasma pneumoniae and Mycoplasma hominis respectively. Antimicrob Agents Chemother, 2005, 49, 3: 1190-1193). Ces problèmes de résistance rendent le diagnostic d'infection à *M. pneumoniae* essentiel pour que l'antibiothérapie soit appropriée et administrée précocement.

A l'heure actuelle, le diagnostic direct est réalisée par culture ou réaction de polymérisation en chaîne (PCR) ; quant au diagnostic indirect, des tests sérologiques existent tels que les agglutinines froides, la réaction de fixation du complément, l'immunofluorescence indirecte, l'agglutination passive et les tests ELISA. Toutefois, il n'existe pas de test de diagnostic de référence pour la détection d'infections à M. *pneumoniae.*

La culture de *M. pneumoniae* peut être réalisée à partir de prélèvements de gorge, d'aspirations nasopharyngées chez l'enfant et de lavages bronchoalvéolaires, mais elle est longue (2 à 3 semaines) et fastidieuse. Rarement pratiquée en routine, elle est plutôt réservée aux laboratoires de référence. Cependant, lorsqu'elle est positive, elle est spécifique à 100%. La persistance de la bactérie pouvant aller jusqu'à plusieurs semaines après l'infection, des tests complémentaires, comme le dosage d'anticorps spécifiques, sont nécessaires pour confirmer le diagnostic d'une infection active.

L'amplification génique par PCR, à partir de prélèvements du tractus respiratoire, est une méthode plus rapide et plus sensible que la culture (sur 100 prélèvements positifs en PCR, seulement 60 sont positifs en culture). Différents systèmes ont été proposés, amplification de séquences au hasard, amplification du gène de l'adhésine ou encore du gène codant l'ARN 16S. Cette approche est sensible (78-92%), permettant la détection de 10 à 100 cfu avec une bonne spécificité (92-100%) (Loens K, Goossens H and leven M., Molecular Diagnosis of Mycoplasma pneumoniae respiratory tract infections, J. Clin. Microbiol., 2003) . Cependant, aucun kit de détection n'est commercialisé, la technique n'étant donc pas standardisée et restant très coûteuse et trop complexe à utiliser en routine dans la plupart des laboratoires de microbiologie clinique.

Les sérologies sont les méthodes les plus utilisées dans le diagnostic d'infection à *M. pneumoniae,* notamment en cas d'absence de prélèvements. A la suite d'une infection à *M. pneumoniae,* le système immunitaire d'un individu non immunodéprimé répond rapidement par la production d'anticorps atteignant un pic au bout de 3 à 6 semaines pour ensuite décliner graduellement sur une période allant de quelques mois à quelques années. La production d'immunoglobulines M (IgM), spécifiques de *M. pneumoniae,* apparaît 7 à 10 jours après le début de l'infection. Leur mise en évidence est souvent la preuve d'une infection récente, surtout chez les jeunes enfants qui n'ont pas eu d'expositions répétées à la bactérie. Cependant, chez l'adulte ayant eu une exposition répétée, l'infection à *M. pneumoniae* ne provoque pas de montée rapide d'IgM et, dans ce cas, les tests sérologiques commercialisés mettant en évidence une réponse IgM peuvent être pris en défaut. De même, la réponse IgM pouvant persister pendant des mois, voire des années, le taux d'anticorps IgM ne reflète pas forcément une infection récente. Dans certains cas, la réinfection conduit à une augmentation du taux d'IgG et c'est pourquoi il est préconisé de rechercher en parallèle une réponse IgG et IgM. Les IgA sont produits tôt au cours de l'infection mais leur niveau de production décroît plus rapidement que celui des IgG et des IgM. Ils peuvent être de bons indicateurs d'une infection récente pour toutes les classes d'âges, même après de multiples réinfections (Waites K.B., C.M. Bébéar, J.A. Robertson, D.F. Talkington, and G.E. Kenny. Laboratory diagnosis of mycoplasmal infections. Cumitech of American Society for Microbiology, coordinating editor : F.S. Nolte., 2001, ASM press : 1-30).

Il existe différentes techniques sérologiques, souvent complémentaires, pour diagnostiquer une infection à *M. pneumoniae.* Les principaux tests sont la recherche d'agglutinines froides, la réaction de fixation du complément, l'immunofluorescence indirecte, les tests d'agglutination passive et les tests ELISA.

La présence d'agglutinines froides, évocatrice à un taux >64, est parfois constatée dans les infections à *M. pneumoniae* mais elle n'est ni constante ni caractéristique. Cette technique autrefois utilisée n'est donc plus recommandée dans la recherche d'infection à *M. pneumoniae.*

La réaction de fixation du complément (RFC), utilisant une préparation antigénique effectuée à partir du micro-organisme entier, a longtemps été utilisée. Le test mesure le taux d'IgM et d'IgG simultanément, sans les différencier. Un titre >64, est évocateur d'une infection. La technique est lourde, peu sensible et peut donner lieu à des réactions croisées ou à des résultats ininterprétables (sérums " anticomplémentaires ").

La recherche d'anticorps spécifiques peut se faire par immunofluorescence indirecte. Le sérum à tester, mis au contact d'antigènes de *M. pneumoniae,* est révélé par des anticorps anti-IgM ou anti-IgG humaines, conjugués à un fluorochrome. Cette technique existe sous forme de kit commercialisé mais nécessite un microscope à fluorescence. La lecture des lames est longue et fastidieuse et l'interprétation des résultats reste délicate.

Des tests d'agglutination passive pour la détection d'IgM et/ou d'IgG sont commercialisés. Ils mettent en évidence une reconnaissance par des anticorps, contenus dans le sérum du patient, d'antigènes (extraits de *M. pneumoniae*) fixés à des particules de latex, de gélatine ou encore d'érythrocytes dans le cas du test d'hémagglutination indirecte (IHA). La technique nécessite au moins deux sérums pour mettre en évidence une augmentation du titre d'anticorps et ne présente pas d'avantages par rapport à la technique ELISA (Waites K.B., C.M. Bébéar, J.A. Robertson, D.F. Talkington, and G.E. Kenny. Laboratory diagnosis of mycoplasmal infections. Cumitech of American Society for Microbiology, coordinating editor : F.S. Nolte, 2001, ASM press : 1-30) .

Les techniques ELISA permettent de détecter indépendamment des IgG ou des d'IgM. Des préparations d'extraits bactériens, de protéines purifiées comme l'adhésine P1, de glycolipides, de peptides synthétiques ont été utilisés, fixés au support solide. Le sérum des patients est incubé avec la phase solide antigénique, et des anticorps anti-IgG ou anti-IgM humaines conjugués à un enzyme réagissent avec les anticorps liés à l'antigène. Le complexe est mis en évidence par l'hydrolyse d'un substrat de l'enzyme aboutissant à un produit coloré. Le choix de l'ELISA (IgM et/ou IgG) dépend de l'âge du patient et du nombre de sérums pouvant être testés. La présence d'IgM est très évocatrice chez l'enfant et l'adolescent mais plus rarement observée chez l'adulte. Il est préférable de rechercher des anticorps spécifiques sur deux sérums prélevés à 10-15 jours d'intervalle, pour mettre en évidence une séroconversion (augmentation x 4 du titre d'anticorps) .

En résumé, les pratiques courantes ne répondent toujours pas aux attentes médicales pour établir de façon certaine le diagnostic d'infections à *M. pneumoniae* chez l'enfant ou l'adulte. Bien que les tests sérologiques semblent être les plus adaptés, dans de nombreux cas le diagnostic d'une infection active reste difficile à différencier de celui d'une infection passée (Waites K.B., C.M. Bébéar, J.A. Robertson, D.F. Talkington, and G.E. Kenny. Laboratory diagnosis of mycoplasmal infections. Cumitech of American Society for Microbiology, coordinating editor : F.S. Nolte., 2001, ASM press : 1-30, Talkington DF, Shott S, Fallon MT, Schwartz SB and Thacker WL. Analysis of eight commercial enzyme immunoassay tests for détection of antibodies to Mycoplasma pneumoniae in human serum, Clin. Diagn. Lab. Immunol., 2004) .

US5788962 décrit l'utilisation du polypeptide P65 ou du polynucléotide codant cette protéine pour détecter dans des échantillons biologiques non humains (solution contenant les membranes des mycoplasmes) la présence des anticorps produits à la suite d'une infection à *Mycoplasma pneumonia.*

SJOSTROM K E ET AL, vol. 33, no. 2, 1983, pages 218-228 décrit, que les ATPases présentent des spécificités sérologiques différentes d'une espèce à l'autre, mais également à l'intérieure d'une même espèce. Ainsi, les ATPases de *M. pneumoniae* ne sont reconnues que par les anticorps obtenus en utilisant *M. pneumonia.* Cependant, la production d'anticorps est obtenue en hyper-immunisant des lapins, ce qui est très différent de l'infection humain naturel.

WO 96/28472 décrit que la sous unité b de la FoFl-ATPase et/ou ses dérivatives, pourraient être utilisé comme antigènes pour les vaccinations.

La présente invention a notamment comme objectifs de résoudre les déficiences des tests sérologiques actuels et de permettre un diagnostic sensible et spécifique des infections à *Mycoplasma pneumoniae.* C'est dans ce cadre que les inventeurs de la présente invention ont identifié, à partir du génome de *M. pneumoniae,* un polypeptide appelé 7F4, correspondant à la sous-unité béta de l'ATPase, de séquence polypeptidique ID SEQ N°2 dont les utilisations possibles, parmi lesquelles celle indiquée ci-dessous, sont décrites ci-après. La protéine 7F4 est une protéine membranaire exposée dans le cytoplasme de la bactérie et impliquée dans l'activité métabolique de *M. pneumoniae.* Elle est également retrouvée chez d'autres micro-organismes, notamment d'autres espèces de mycoplasmes telles que *M. hyopneumoniae.*

### Définitions

Les définitions suivantes sont données afin de faciliter la compréhension de certains termes utilisés dans cette description.

On entend par "polynucléotide", un polyribonucléotide ou un polydésoxyribonucléotide qui peut être un ADN ou un ARN modifié ou non.

Le terme polynucléotide inclut, sans limitation, un ADN simple brin ou double brin, un ADN composé d'un mélange d'une ou plusieurs région(s) simple brin et d'une ou plusieurs région(s) double brin, un ADN qui est un mélange de régions simple brin, double brin et/ou triple brin, un ARN simple brin ou double brin, un ARN composé d'un mélange d'une ou plusieurs région(s) simple brin et d'une ou plusieurs région(s) double brin et les molécules hybrides comprenant un ADN et un ARN qui peuvent comprendre des régions simple brin, double brin et/ou triple brin ou un mélange de régions simple brin et double brin. Le terme polynucléotide peut aussi comprendre un ARN et/ou un ADN comprenant une ou plusieurs régions triple brin. Les brins dans de telles régions peuvent provenir de la même molécule ou de molécules différentes. Par conséquent les ADN ou ARN, ayant des squelettes modifiés pour la stabilité, ou d'autres raisons, sont inclus dans le terme polynucléotides. On entend également par polynucléotide, les ADNs et ARNs contenant une ou plusieurs bases modifiées. On entend par "base modifiée", par exemple, les bases inhabituelles telles que l'inosine. Le terme polynucléotide vise également les polynucléotides de forme modifiée chimiquement, enzymatiquement ou métaboliquement. Les polynucléotides comprennent également les polynucléotides courts tels que les oligonucléotides.

On entend par "polypeptide", un peptide, un oligopeptide, un oligomère ou une protéine comprenant au moins deux acides aminés joints l'un à l'autre par une liaison peptidique normale ou modifiée.

Le terme polypeptide comprend les chaînes courtes, appelées peptides, oligopeptides et oligomères, et les chaînes longues, appelées protéines.

Un polypeptide peut être composé d'acides aminés autres que les 20 acides aminés codés par les gènes humains. Un polypeptide peut également être composé d'acides aminés modifiés par des processus naturels, tels que le processus de maturation post-traductionnel ou par des procédés chimiques, qui sont bien connus de l'homme du métier. Le même type de modification peut être présent à plusieurs endroits du polypeptide et n'importe où dans le polypeptide : dans le squelette peptidique, dans la chaîne d'acides aminés ou encore aux extrémités carboxy- ou amino-terminales.

Un polypeptide peut être ramifié suite à une ubiquitination ou être cyclique avec ou sans ramification. Ce type de modifications peut être le résultat de processus de post-traduction naturels ou synthétiques, qui sont bien connus de l'homme du métier.

On entend, par exemple, par modifications d'un polypeptide, l'acétylation, l'acylation, l'ADP-ribosylation, l'amidation, la fixation covalente de flavine, la fixation covalente d'un hème, la fixation covalente d'un nucléotide ou d'un dérivé nucléotidique, la fixation covalente d'un lipide ou d'un dérivé lipidique, la fixation covalente d'un phosphatidylinositol, la réticulation covalente ou non-covalente, la cyclisation, la formation de pont disulfure, la déméthylation, la formation de cystéine, la formation de pyroglutamate, la formylation, la gamma-carboxylation, la glycosylation, la formation d'ancre au GPI, l'hydroxylation, l'iodisation, la méthylation, la myristoylation, l'oxydation, le processus protéolytique, la phosphorylation, la prénylation, la racémisation, la sénéloylation, la sulfatation, l'addition d'acides aminés, telle que l'arginylation ou l'ubiquitination . (PROTEINS STRUCTURE AND MOLECULAR PROPERTIES, 2nd Ed., T.E. Creighton, W.H. Freeman and Company, New York (1993) and Wold, F., Posttranslational Protein Modifications: Perspectives and Prospects, pgs 1-12 in POSTTRANSLATIONAL COVALENT MODIFICATION OF PROTEINS, B.C. Johnson, Ed., Academic Press, New York (1983); Seifter et al., Meth. Enzymol. 182: 626-646 (1990) and Rattan et al., Protein Synthesis : Posttranslational Modifications and Aging, Ann. N.Y. Acad. Sci. 663 : 48-62 (1992)).

On entend par "isolé", modifié par la main de l'homme à partir de l'état naturel, c'est-à-dire que le polynucléotide ou le polypeptide présent dans la nature a été modifié ou isolé de son environnement naturel, ou les deux. Par exemple, un polynucléotide ou un polypeptide naturellement présent dans un organisme vivant n'est pas "isolé", mais le même polynucléotide ou polypeptide séparé des matériaux coexistants dans son état naturel est "isolé".

On entend par "pourcentage d'identité" entre deux séquences polynucléotidiques ou polypeptidiques le pourcentage de nucléotides ou d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons entre deux séquences polynucléotidiques ou polypeptidiques sont traditionnellement réalisées en comparant ces séquences après les avoir alignées de manière optimale, ladite comparaison étant réalisée par segment ou par "fenêtre de comparaison" pour identifier et comparer les régions locales de similarité de séquence. Cette comparaison peut être réalisée grâce à un programme, par exemple le programme EMBOSS-Needle (alignement global Needleman-Wunsch) à l'aide de la matrice BLOSUM62/Open Gap 10.0 et Extension Penalty de 0,5 (Needleman, S.B. and Wunsch, C.D. (1970), J. Mol. Biol. 48, 443-453 et Kruskal, J.B. (1983), An overview of sequence comparison, In D. Sankoff and J.B. Kruskal, (ed), Time warps, strind edits and macromolecules : the theory and practice of sequence comparison, pp. 1-44 Addison Wesley) .

Le pourcentage d'identité est calculé en déterminant le nombre de positions identiques pour lesquelles le nucléotide ou l'acide aminé est identique entre les deux séquences, en divisant ce nombre de positions identiques par le nombre total de positions dans la fenêtre de comparaison et en multipliant le résultat obtenu par 100.

Un polypeptide ayant, par exemple, une identité d'au moins 95% avec le polypeptide ID SEQ N°2 est un polypeptide comportant, au plus, 5 acides aminés modifiés sur 100 acides aminés, par rapport à ladite séquence. En d'autres termes jusqu'à 5% des acides aminés dans la séquence ID SEQ N°2 peuvent être délétés ou substitués par un autre acide aminé ou la séquence peut comporter jusqu'à 5% d'acides aminés en plus par rapport au nombre total d'acides aminés de la séquence ID SEQ N°2. Ces altérations de la séquence peuvent être situées aux positions amino-et/ou carboxy-terminales de la séquence d'acides aminés ou à un endroit quelconque entre ces positions terminales, en un ou plusieurs emplacements. (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987) .

Par analogie, un polynucléotide ayant, par exemple, une identité d'au moins 95% avec le polynucléotide ID SEQ N°1 est donc un polynucléotide comportant, au plus, 5 nucléotides modifiés sur 100 nucléotides, par rapport à la séquence dudit polynucléotide. En d'autres termes jusqu'à 5% des nucléotides du polynucléotide ID SEQ N°1 peuvent être délétés ou substitués par un autre nucléotide, ou un polynucléotide peut comporter jusqu'à 5% de nucléotides en plus par rapport au nombre total de nucléotides du polynucléotide ID SEQ N°1. Ces modifications peuvent être positionnées aux extrémités 3' et/ou 5', ou à un endroit quelconque entre ces extrémités, en un seul ou plusieurs emplacements.

On entend par "fragment de polypeptide" un polypeptide comprenant au moins "n" acides aminés consécutifs issus du polypeptide ID SEQ N°2, où, selon les séquences, n sera égal à 7 acides aminés ou plus (par exemple, 8, 10, 12, 14, 16, 18, 20, 30, 40, 50 ou plus acides aminés). Préférentiellement, lesdits fragments comportent un épitope de la séquence ID SEQ N°2. Ces épitopes de type B ou T peuvent être déterminés par un logiciel (par exemple, PEOPLE [Alix, 1999] , PREDITOP [Pellequer et Westhof, 1993] ou TEST [Zao et al., 2001]) ou expérimentalement par des techniques classiques [par exemple, épitope mapping (cartographie d'épitope) ou protéolyse ménagée].

On entend par "fragment de polynucléotide" un polynucléotide comprenant au moins "n" nucléotides consécutifs issus du polynucléotide ID SEQ N°1, où, selon les séquences, n sera égal à 21 nucléotides ou plus (par exemple, 22, 23, 24, 25, 30, 36, 42, 48, 54, 60, 90, 120, 150 ou plus nucléotides).

On entend par "cellule hôte", une cellule qui a été transformée ou transfectée, ou est capable de transformation ou de transfection, par une séquence polynucléotidique exogène.

On entend par "amorces spécifiques" de courtes séquences nucléotidiques capables de s'hybrider de façon spécifique, grâce à la complémentarité des bases, sur le brin d'ADN ou sur son brin complémentaire.

On entend par "milieu de culture", le milieu dans lequel on purifie le polypeptide de l'invention. Ce milieu peut être constitué par le milieu extracellulaire et/ou le lysat cellulaire. Des techniques bien connues de l'homme du métier permettent également à ce dernier de redonner la conformation active au polypeptide, si la conformation dudit polypeptide a été altérée lors de l'isolation ou de la purification.

On entend par "fonction", l'activité biologique d'un polypeptide ou d'un polynucléotide. La fonction d'un polypeptide conforme à l'invention est celle d'un antigène de M. *pneumoniae,* et la fonction d'un polynucléotide conforme à l'invention est celle de coder ce polypeptide.

On entend par "antigène", tout composé qui, seul ou en association avec un adjuvant ou porteur, est capable d'induire une réponse immunitaire spécifique. Cette définition comprend également tout composé présentant une analogie structurale avec ledit antigène capable d'induire une réponse immunologique dirigée contre ledit antigène.

On entend par "analogie structurale" une analogie aussi bien de la structure primaire (séquence) que de la structure secondaire (éléments structuraux), de la structure tertiaire (structure tridimensionnelle) ou de la structure quaternaire (association de plusieurs polypeptides dans un même complexe) (BIOCHEMISTRY, 4ème Ed, L. Stryer, New York, 1995).

On entend par "variant" d'un polynucléotide dit initial ou d'un polypeptide dit initial, respectivement, un polynucléotide ou un polypeptide qui en diffère par au moins un nucléotide ou un acide aminé, mais qui garde les mêmes propriétés intrinsèques, c'est-à-dire la même fonction.

Une différence dans la séquence polynucléotidique du variant peut altérer ou non la séquence d'acides aminés du polypeptide qu'il code, par rapport à un polypeptide initial. Néanmoins, par définition, ces variants doivent conférer la même fonction que la séquence polynucléotidique initiale, par exemple, coder un polypeptide ayant une fonction antigénique.

Le polynucléotide ou le polypeptide variant diffère généralement du polynucléotide initial ou du polypeptide initial par une (ou plusieurs) substitution(s), addition(s), délétion(s), fusion(s) ou troncation(s) ou plusieurs de ces modifications, prises en combinaison. Un variant non-naturel d'un polynucléotide initial ou d'un polypeptide initial peut être obtenu, par exemple, par mutagenèse dirigée ou par synthèse directe.

On définit comme "séquence polynucléotidique complémentaire à la séquence polynucléotidique", un polynucléotide qui peut être hybridé avec cette séquence polynucléotidique dans des conditions stringentes.

On entend généralement, mais pas nécessairement, par "conditions stringentes" les conditions chimiques qui permettent une hybridation lorsque les séquences polynucléotidiques ont une identité d'au moins 80%. Ces conditions peuvent être obtenues selon les méthodes bien connues de l'homme du métier.

On entend par "anticorps", les anticorps monoclonaux, polyclonaux, chimériques, simple chaîne, humanisés, ainsi que les fragments Fab, incluant les produits d'un Fab ou d'une banque d'expression d'immunoglobulines.

Un anticorps immunospécifique peut être obtenu par administration à un animal d'un polypeptide donné, suivie d'une récupération des anticorps produits par ledit animal par extraction depuis ses fluides corporels. On peut également administrer à l'animal un variant dudit polypeptide, ou des cellules hôtes exprimant ce polypeptide.

Le terme "immunospécifique" appliqué au terme anticorps, vis-à-vis d'un polypeptide donné, signifie que l'anticorps possède une meilleure affinité pour ce polypeptide que pour d'autres polypeptides connus de l'art antérieur.

Par "affinité", on entend à la fois une complémentarité structurale et une complémentarité des liaisons de faible énergie entre deux molécules au sens d'une définition communément admise (voir, par exemple, Klotz IM. Ligand-protein binding affinities. In Protein Function, a Practical Approach (T. E. Creighton, Ed.). 1989; 25-35. IRL Press, Oxford, ou encore Ajay, Murcko MA. Computational methods to predict binding free energy in ligand-receptor complexes.J Med Chem. 1995 ; 38:4953-67). L'affinité peut être mesurée par les techniques classiques de la biochimie (ELISA, compétition, fluorescence...) bien connues de la personne du métier.

Par sérum "positif" on entend un sérum contenant des anticorps, produits à la suite d'une infection à *M. pneumoniae,* identifiés par leur liaison avec le polypeptide (antigène) de l'invention.

On entend par "sensibilité" la proportion de malades infectés, diagnostiqués selon l'art antérieur et donnés positifs par le diagnostic selon l'invention.

On entend par "spécificité" la proportion de donneurs de sang, testés comme témoins, soumis au diagnostic selon l'invention et donnés négatifs par le diagnostic selon l'invention.

On entend par "kit de diagnostic" un ensemble contenant au moins l'un des produits selon l'invention (polypeptide, polynucléotide) et un diluant convenable, rassemblés dans un contenant approprié fait dans un matériau adapté. Ce contenant peut rassembler les différents moyens complémentaires nécessaires au test sérologique (par exemple, des réactifs marqués, des tampons, des solutions qui contiennent des ions adaptés, etc...) ainsi que les instructions requises pour réaliser le test.

Comme indiqué plus haut, l'invention a pour objet l'utilisation dans le domaine du diagnostic *in vitro* d'infections à *M. pneumoniae* et/ou dans la production de vaccins contre *M. pneumoniae,* de polypeptides selon l'invention, de polynucléotides codant pour lesdits polypeptides, de vecteurs d'expression comprenant lesdits polynucléotides, de cellules hôtes comprenant lesdits vecteurs d'expression.

### Utilisation de polypeptides

L'identification du polypeptide selon l'invention est le résultat d'un crible et d'études approfondies que les séquences issues des programmes de génomique de *M. pneumoniae* ne laissaient pas envisager.

La présente invention a ainsi pour objet l'utilisation d'un polypeptide isolé comprenant la séquence d'acides aminés ID SEQ N°2 (appelée protéine 7F4), codée
par la séquence polynucléotidique ID SEQ N°1, pour détecter, *in vitro*, dans des échantillons biologiques issus d'un être humain, la présence d'anticorps produits à la suite d'une infection à *Mycoplasma pneumonie.* La présente invention vise également l'utilisation d'un polypeptide isolé comprenant :
a) un fragment de la séquence d'acides aminés ID SEQ N°2 ayant la même fonction que la séquence ID SEQ N°2, ou
b) une séquence d'acides aminés ayant au moins 60% d'identité, de préférence au moins 80% d'identité, et mieux au moins 90% d'identité, avec la séquence d'acides aminés ID SEQ N°2, ou avec le fragment de séquence défini sous a), et ayant la même fonction que la séquence ID SEQ N°2, étant entendu que :
   par "fragment de la séquence d'acides aminés", on entend un polypeptide comprenant au moins "n" acides aminés consécutifs issus du polypeptide ID SEQ N°2, où, selon les séquences, n sera égal à 7 acides aminés ou plus, et
   par "ayant la même fonction que ladite séquence ID SEQ N°2", on entend une fonction d'antigène de *Mycoplasma pneumoniae.*

La présente invention divulgue également un procédé de préparation d'un polypeptide tel que défini ci-dessus, dans lequel une cellule hôte définie précédemment est cultivée et ledit polypeptide est isolé du milieu de culture.

Le polypeptide peut être purifié à partir des cellules hôtes, selon les méthodes bien connues de l'homme du métier telles que la précipitation à l'aide d'agents chaotropiques comme les sels, en particulier le sulfate d'ammonium, l'éthanol, l'acétone ou l'acide trichloroacétique, ou de moyens tels que l'extraction à l'acide, la chromatographie échangeuse d'ions, la chromatographie sur phosphocellulose, la chromatographie par interaction hydrophobe, la chromatographie d'affinité, la chromatographie sur hydroxylapatite ou les chromatographies d'exclusion.

### Utilisation de polynucléotides

La présente invention a notamment pour objet l'utilisation pour détecter *in vitro,* dans des échantillons biologiques issus d'un être humain, la présence d'anticorps produits à la suite d'une infection à *Mycoplasma pneumoniae,* d'un polynucléotide isolé comprenant la séquence polynucléotidique ID SEQ N°1, codant pour le polypeptide comprenant la séquence d'acides aminés ID SEQ N°2. L'invention vise également l'utilisation d'un polynucléotide isolé comprenant :
a) un fragment de la séquence ID SEQ N°1 ayant la même fonction que la séquence ID SEQ N°1, ou
b) une séquence polynucléotidique ayant au moins 60% d'identité, de préférence au moins 80% d'identité, et mieux au moins 90% d'identité, avec la séquence polynucléotidique ID SEQ N°1, ou avec le fragment de séquence tel que défini sous a), et ayant la même fonction que la séquence ID SEQ N°1, ou
c) une séquence polynucléotidique complémentaire à la séquence polynucléotidique ID SEQ N°1 ou au fragment de séquence défini sous a) ou à la séquence définie sous b), étant entendu que :
   par "fragment de la séquence du polynucléotide" on entend un polynucléotide comprenant au moins "n" nucléotides consécutifs issus du polynucléotide ID SEQ N°1, où, selon les séquences, n sera égal à 21 nucléotides ou plus, et
   par "ayant la même fonction que ladite séquence ID SEQ N°1", on entend une fonction de codage d'un polypeptide antigène de *Mycoplasma pneumoniae.*

Ainsi, des polynucléotides conformes à l'invention peuvent comprendre des variants ou des fragments de la séquence polynucléotidique ID SEQ N°1.

Les polynucléotides de l'invention peuvent être obtenus par les méthodes standard de synthèse d'ADN ou d'ARN.

Les polynucléotides conformes à l'invention peuvent également comprendre des séquences polynucléotidiques comme les séquences 5' et/ou 3' non-codantes, telles que, par exemple, des séquences transcrites, des séquences non-traduites, des séquences signal d'épissage, des séquences polyadénylées, des séquences de liaison avec des ribosomes ou encore des séquences qui stabilisent l'ARNm.

### Utilisation de vecteurs d'expression et de cellules hôtes

La présente invention a aussi pour objet l'utilisation du polypeptide selon l'invention préparé par culture d'une cellule hôte comprenant un vecteur recombinant ayant, inséré, un polynucléotide codant pour ledit polypeptide conforme à l'invention.

De nombreux systèmes d'expression peuvent être utilisés comme, par exemple, les chromosomes, les épisomes, les virus dérivés. Plus particulièrement, les vecteurs recombinants utilisés peuvent être dérivés de plasmides bactériens, de transposons, d'épisome de levure, d'éléments d'insertion, d'éléments chromosomiques de levures, de virus tels que les *baculovirus,* les *papillonna virus* comme SV40, les *vaccinia virus,* les adénovirus, les *fox pox virus,* les *pseudorabies virus,* les rétrovirus.

Ces vecteurs recombinants peuvent également être des dérivés de cosmides ou de phagemides. La séquence polynucléotidique peut être insérée dans le vecteur recombinant d'expression par les méthodes bien connues de l'homme du métier.

Le vecteur recombinant peut comprendre des séquences polynucléotidiques de contrôle de la régulation de l'expression du polynucléotide ainsi que des séquences polynucléotidiques permettant l'expression et la transcription d'un polynucléotide de l'invention et la traduction d'un polypeptide de l'invention, ces séquences étant choisies en fonction des cellules hôtes mises en oeuvre.

L'introduction du vecteur recombinant dans une cellule hôte peut être effectuée selon les méthodes bien connues de l'homme du métier telles que la transfection par phosphate de calcium, la transfection par lipides cationiques, l'électroporation, la transduction ou l'infection.

Les cellules hôtes peuvent être, par exemple, des cellules bactériennes telles que des cellules de streptocoques, de staphylocoques, d'*Escherichia coli* ou de *Bacillus subtilis,* des cellules de champignons telles que des cellules de levure et des cellules d'*Aspergillus,* des cellules de *Streptomyces,* des cellules d'insectes telles que des cellules de *Drosophilia S2* et de *Spodoptera Sf9,* des cellules animales, telles que les cellules CHO, COS, HeLa, C127, BHK, HEK 293 ou encore des cellules végétales.

Le polypeptide peut être purifié à partir des cellules hôtes, selon les méthodes bien connues de l'homme du métier, telles que la précipitation à l'aide d'agents chaotropiques comme les sels, en particulier le sulfate d'ammonium, l'éthanol, l'acétine ou l'acide trichloroacétique, ou de moyens tels que l'extraction à l'acide, la chromatographie échangeuse d'ions, la chromatographie sur phosphocellulose, la chromatographie par interaction hydrophobe, la chromatographie d'affinité, la chromatographie sur hydroxylapatite ou les chromatographies d'exclusion.

### Sérologie

Les échantillons biologiques testés peuvent être du sang, de l'urine, de la salive, du liquide de ponction de sérologie (par exemple liquide céphalo-rachidien, liquide pleural ou liquide articulaire) ou l'un de leurs constituants (par exemple, le sérum).

### Les vaccins

La présente invention concerne également un vaccin pour la prévention des infections à Mycoplasma pneumoniae chez l'être humain

renfermant à titre de principe actif au moins un des polypeptides du polynucléotides selon l'invention, et un excipient pharmaceutiquement acceptable (par exemple, une solution stérile aqueuse ou non, pouvant contenir un antioxydant ou un tampon ou un soluté rendant la composition isotonique pour les fluides corporels).

### Les kits

L'invention divulgue également des kits de diagnostic in vitro comprenant, d'une part :
- au moins l'un des polypeptides conformes à l'invention, ou
- au moins l'un des polynucléotides codant pour lesdits polypeptides,
et, d'autre part, au moins un diluant (par exemple, un tampon, une solution saline...) et une notice d'instructions d'utilisation.

### Partie expérimentale

### A) Protocole d'obtention des antigènes

### A.1) Clonage de la séquence codant pour le polypeptide ID SEQ N°2

Le gène codant pour la séquence du polypeptide 7F4 selon l'invention, qui est antigénique, est obtenu par amplification par PCR à partir de l'ADN génomique de la bactérie *M. pneumoniae* (souche M129-B7, ATCC 29342) en utilisant comme couple d'amorce :
l'oligonucléotide sens contenant la séquence :
   5'-AAAAAGGAAAACATTACATACG -3' ; et
l'oligonucléotide antisens contenant la séquence :
   5'- TTTCTCCTCAACAGTAG-3'.

Le fragment correspondant, ainsi amplifié, est cloné dans un vecteur selon les techniques classiques bien connues de l'homme du métier. Ce vecteur permet la production de la protéine clonée sous contrôle d'un promoteur inductible par l'isopropyl-thiogalactoside (en abrégé, IPTG). La protéine clonée correspond à la séquence d'acides aminés ID SEQ N°2.

### A.2) Expression de la protéine

Une souche d'*Escherichia coli* est transformée par le vecteur d'expression précédemment décrit. Les bactéries sélectionnées sont mises en culture une nuit à 30°C sous agitation dans 50 ml de milieu Luria Bertani (LB, J.Miller, « A short course in Bacteria Genetics », Cold Spring Harbor Laboratory Press, 1992) contenant de l'ampicilline et du chloramphénicol tous deux à une concentration finale de 100 µg/ml. Le lendemain, la culture est diluée au 1/50^{ème} dans un volume final de 1 litre de milieu LB auquel on a ajouté ampicilline et chloramphénicol tous deux à une concentration finale de 100 µg/ml et incubée à 30°C sous agitation. Lorsque la turbidité de la culture atteint une valeur d'absorbance à 600 nm (en abrégé, A600) d'environ 0,7, la production de la protéine est induite par l'isopropyl-thiogalactoside (IPTG) à une concentration finale de 0,5 mM. Les bactéries sont récoltées par centrifugation (6 minutes à 5240 rpm à 4°C) lorsque la turbidité de la culture atteint une A600 d'environ 1,5.

### A.3) Purification

Après centrifugation, les cellules sont remises en suspension dans un tampon Tris-HCl 20 mM pH 8 contenant du sucrose à 0,5 mM, puis traitées avec du lysozyme (0,1 g/50 ml) en présence d'acide éthylènediaminotétraacétique (EDTA) 250 mM. La suspension est incubée 30 minutes à 4°C puis centrifugée 10 minutes à 4°C à 15500x g. Le culot est congelé à -20°C pendant au moins une nuit.

Après décongélation, les bactéries sont reprises dans : un tampon Mes [acide 2-(N-morpholino)éthane sulfonique] 25 mM à pH 6,0, puis soniquées 4 fois 20 secondes dans la glace. Après centrifugation à 15500 g à 4°C pendant 30 minutes, les culots sont repris dans 10 ml de tampon Mes 25 mM à pH 6,0, urée 8 M, β mercaptoéthanol 20 mM). Puis la suspension est centrifugée 20 minutes à 14 000 rpm à température ambiante (TA). Le surnageant est à nouveau centrifugé 30 minutes à TA à 14000 rpm, puis filtré sur membrane de porosité 0,22 µm. Le filtrat est alors déposé sur une colonne échangeuse de cations (par exemple, SP-Sépharose 12 ml, Amersham Biosciences). Après lavage de la colonne, la protéine est éluée par un gradient linéaire de 0 à 1 M de NaCl dans du tampon Mes 25 mM à pH 6,0, urée 8 M, β mercaptoéthanol 20 mM en 20 volumes de colonne. Les fractions contenant la protéine sont rassemblées, dialysées sur la nuit contre du tampon Mes 25 mM à pH 6,0, urée 8 M, β mercaptoéthanol 20 mM puis redéposé sur cette même colonne. La protéine est ensuite éluée par un nouveau gradient de NaCl optimisé pour la protéine 7F4. Les fractions choisies contenant la protéine sont rassemblées puis dialysées sur la nuit contre du tampon Na₂HPO₄/NaHPO₂ 50 mM pH 8,0 contenant du NaCl 100 mM puis déposées sur une colonne de gel de filtration (par exemple, Superdex HR75-10/30, Amersham). Les fractions éluées contenant la protéine sont rassemblées et conservées à température ambiante jusqu'à leur utilisation dans les tests.

Les concentrations protéiques sont déterminées à partir des coefficients d'absorption molaire calculés par la méthode de Pace (Pace CN, Vajdos F., Fee L., Grismley G. et Gray T., (1995), Protein Science 4, 2411-2423). La pureté des protéines est vérifiée par analyse par électrophorèse SDS-PAGE.

### B) Test de diagnostic in vitro

Il a été utilisé des sérums provenant de patients enfants et adultes ayant eu une infection documentée à *M. pneumoniae* (collection du laboratoire ; c'est-à-dire titre IgM et/ou IgG et/ ou RFC positifs et/ou PCR positif et /ou culture positive).

Les sérums témoins correspondent à des sérums de donneurs de sang (collection du laboratoire).

La fixation, sur la protéine recombinante purifiée 7F4 (obtenue comme décrit précédemment), des anticorps présents dans les sérums a été évaluée soit par des tests en Western Blot soit par la technique ELISA.

### Exemple B.1 : Protocole de test pour les polypeptides selon l'invention en Western Blot

Après transfert sur une membrane de nitrocellulose de la protéine purifiée 7F4, la membrane est saturée pendant 45 minutes à l'aide d'une solution de tampon salin phosphate (PBS) contenant 3% de lait demi-écrémé. Après trois lavages par du PBS contenant du polyoxyéthylène sorbitan (Tween) à 0,05%, la membrane est mise en présence du sérum testé à la dilution adéquate (1/500) dans du tampon PBS contenant 3% de lait demi-écrémé pendant 45 minutes. Après trois nouveaux lavages, des anticorps anti-immunoglobulines G et A et M humaines de chèvre (par exemple 170 -1042, Biorad) marqués à la phosphatase alcaline sont ajoutés pendant une période de 45 minutes après avoir été dilués, selon le protocole du fournisseur, dans du tampon PBS contenant 3% de lait demi-écrémé. Trois nouveaux lavages sont effectués puis du phosphate de 5-bromo-4-chloro-3-indolyle et du nitroblue tetrazolium sont ajoutés selon les indications du fournisseur jusqu'à l'obtention du résultat. Un résultat " positif " correspond à la fixation de l'anticorps anti-immunoglobuline humaine à un complexe composé du polypeptide selon l'invention fixé à la membrane et de l'anticorps sérique humain le reconnaissant spécifiquement, ce qui se traduit par une coloration localisée au niveau dudit complexe.

### Exemple B.2 : Protocole du test ELISA

Les plaques ELISA sont laissées pendant une nuit à température ambiante en présence de 0,5 µg d'antigène purifié (protéine 7F4 recombinante) dans du tampon salin phosphate (PBS) - urée 6 M. Après quatre lavages par du PBS contenant du polyoxyéthylène sorbitan (Tween) à 0,05%, les plaques sont saturées une heure et demie à 37°C par du PBS-Tween contenant 4% d'albumine de sérum bovin (BSA) (250 µl par puits). Quatre nouveaux lavages sont réalisés puis 100 µl de chaque sérum à tester sont ajoutés à la dilution 1/100 dans du tampon PBS-BSA 4% dans chaque puits. La plaque est ensuite incubée à 37°C pendant 30 minutes. Après quatre nouveaux lavages, des anticorps anti-immunoglobulines G et/ou A et/ ou M humaines marqués à la peroxydase (par exemple 31415, Pierce) sont ajoutés (simultanément et / ou indépendamment) pendant 30 minutes à 37°C après avoir été dilués, selon le protocole du fournisseur, dans du tampon PBS-BSA 4%. Quatre nouveaux lavages sont réalisés puis 100 µl de substrat tétrabenzimidine (TMB) (par exemple HD979505, Pierce) sont ajoutés par puits. Après incubation pendant environ 15 minutes, 100 µl d'acide sulfurique est ajouté dans chaque puits. L'absorbance à 450 nm de chaque puits est alors mesurée après 5 minutes.

Sont considérés comme " positif " en ELISA, les sérums identifiés par leur liaison avec les polypeptides (antigènes) de l'invention.

### C) Résultats et interprétations

Un résultat type obtenu est présenté dans le tableau ci-après. Les sérums de patients (enfants et/ou adultes) considérés comme positifs sont ceux contenant des anticorps dirigés contre *M. pneumoniae,* identifiés par ELISA par leur liaison avec les polypeptides (antigènes) de l'invention.

Tableau de résultats (ELISA) obtenus chez des patients adultes et/ou enfants, en utilisant des anti-IgM comme anticorps secondaires

| | |
|---|---|
| Nombre de sérums " positifs " parmi les 62 sérums de patients (enfants et adultes) infectés par *M. pneumoniae* diagnostiqués selon l'art antérieur et soumis au diagnostic selon l'invention | 32 (soit 51.6%) |
| Nombre de sérums " positifs " parmi les 34 sérums de patients enfants infectés par *M*. *pneumoniae* diagnostiqués selon l'art antérieur et soumis au diagnostic selon l'invention | 24 (soit 70, 6%) |
| Nombre de sérums " positifs " parmi les 28 sérums de patients adultes infectés par *M. pneumoniae* diagnostiqués selon l'art antérieur et soumis au diagnostic selon l'invention | 8 (soit 28,5%) |
| Nombre de sérums " négatifs " parmi les 96 sérums de donneurs de sang témoins | 92 (soit 95,8%) |

Des résultats significatifs peuvent également être obtenus en utilisant des anticorps anti-IgA comme anticorps secondaires. De même des résultats similaires peuvent être obtenus par Western Blot.

D'après le tableau de résultats, on constate que, par le test ELISA, il est possible d'identifier *in vitro* au moins 51,6 % des infections à *M. pneumoniae* quel que soit l'âge du patient, au moins 70,6% des infections à *M. pneumoniae* chez des patients enfants, et au moins 28,5% des infections à *M. pneumoniae* chez des patients adultes grâce à l'antigène selon l'invention.

Il est donc démontré, d'une part, l'existence chez l'homme d'une réponse anticorps significative (la probabilité associée à un test de χ2 est inférieure à 0,05) vis-à-vis de la protéine 7F4 au cours des infections à *M. pneumoniae* et, d'autre part, que la protéine 7F4 est pertinente pour le diagnostic sérologique de ce type d'infection, en particulier chez les enfants.

Un taux important d'anticorps anti-7F4 est constaté chez la plupart des enfants testés. Cet antigène peut donc conférer aux individus infectés une protection immunitaire durable contre la réinfection. L'antigène 7F4 pourrait donc être utilisé pour la vaccination en prévention des infections à *M*. *pneumoniae.*

## Revendications

1. Utilisation du polypeptide de séquence d'acides aminés ID SEQ N°2 pour détecter, *in vitro,* dans des échantillons biologiques issus d'un être humain, la présence d'anticorps produits à la suite d'une infection à *Mycoplasma pneumoniae.*

2. Utilisation pour détecter, *in vitro,* dans des échantillons biologiques issus d'un être humain, la présence d'anticorps produits à la suite d'une infection à *Mycoplasma pneumonie,* d'un polypeptide comprenant :
a) un fragment de la séquence d'acides aminés du polypeptide identifié dans la revendication 1 et ayant la même fonction que ladite séquence ID SEQ N°2, ou
b) une séquence d'acides aminés ayant au moins 60% d'identité avec la séquence d'acides aminés du polypeptide identifié dans la revendication 1 ou avec un fragment de séquence identifié sous a), et ayant la même fonction que ladite séquence ID SEQ N°2,
étant entendu que :
par "fragment de la séquence d'acides aminés", on entend un polypeptide comprenant au moins "n" acides aminés consécutifs issus du polypeptide ID SEQ N°2, où, selon les séquences, n sera égal à 7 acides aminés ou plus, et
par "ayant la même fonction que ladite séquence ID SEQ N°2", on entend une fonction d'antigène de *Mycoplasma pneumoniae.*

3. Utilisation selon la revendication 1 ou 2, **caractérisée en ce que** le polypeptide est préparé par culture d'une cellule hôte comprenant un vecteur recombinant ayant, inséré, un polynucléotide codant pour ledit polypeptide et par isolement dudit polypeptide du milieu de culture.

4. Utilisation selon la revendication 3, quand elle dépend de la revendication 1, **caractérisée en ce que** le polynucléotide est de séquence ID SEQ N°1.

5. Utilisation d'un polynucléotide de séquence ID SEQ N°1 pour détecter, *in vitro,* dans des échantillons
biologiques issus d'un être humain, la présence d'anticorps produits à la suite d'une infection à *Mycoplasma pneumoniae.*

6. Utilisation pour détecter, *in vitro,* dans des échantillons biologiques issus d'un être humain, la présence d'anticorps produits à la suite d'une infection à *Mycoplasma pneumoniae,* d'un polynucléotide comprenant :
a) un fragment de la séquence du polynucléotide identifié dans la revendication 5 et ayant la même fonction que ladite séquence ID SEQ N°1, ou
b) une séquence polynucléotidique ayant au moins 60% d'identité avec la séquence du polynucléotide identifié dans la revendication 5 ou avec un fragment de séquence identifié sous a), et ayant la même fonction que ladite séquence ID SEQ N°1, ou
c) une séquence polynucléotidique complémentaire à une des séquences polynucléotidiqués identifiées dans la revendication 5 ou sous les points a) ou b) ci-dessus,
étant entendu que :
par "fragment de la séquence du polynucléotide" on entend un polynucléotide comprenant au moins "n" nucléotides consécutifs issus du polynucléotide ID SEQ N°1, où, selon les séquences, n sera égal à 21 nucléotides ou plus, et
par "ayant la même fonction que ladite séquence ID SEQ N°1", on entend une fonction de codage d'un polypeptide antigène de *Mycoplasma pneumoniae.*

7. Vaccin pour la prévention des infections à *Mycoplasma pneumoniae* chez l'être humain, comprenant, à titre de principe actif, au moins :
a) un polypeptide de séquence d'acides aminés ID SEQ N°2, ou
b) un polypeptide dont la séquence est un fragment de la séquence d'acides aminés du polypeptide identifié sous a) et ayant la même fonction que ladite séquence ID SEQ N°2, ou
c) un polypeptide dont la séquence est une séquence d'acides aminés ayant au moins 60% d'identité avec la séquence d'acides aminés du polypeptide identifié sous a) ou avec le fragment de séquence identifié sous b), et ayant la même fonction que ladite séquence ID SEQ N°2,
ainsi qu'un excipient pharmaceutiquement acceptable, et étant entendu que :
par "fragment de la séquence d'acides aminés" on entend un polypeptide comprenant au moins "n" acides aminés consécutifs issus du polypeptide ID SEQ N°2, où, selon les séquences, n sera égal à 7 acides aminés ou plus, et
par "ayant la même fonction que ladite séquence ID SEQ N°2", on entend une fonction d'antigène de *Mycoplasma pneumoniae.*

8. Vaccin pour prévention des infections à *Mycoplasma pneumoniae* chez l'être humain, comprenant, à titre.de principe actif, au moins :
a) un polynucléotide de séquence ID SEQ N°1, ou
b) un polynucléotide dont la séquence est un fragment de la séquence du polynucléotide identifié sous a) et ayant la même fonction que ladite séquence ID SEQ N°1, ou
c) un polynucléotide dont la séquence est une séquence polynucléotidique ayant au moins 60% d'identité avec la séquence du polynucléotide identifié sous a) ou avec un fragment de séquence identifié sous b), et ayant la même fonction que ladite séquence ID SEQ N°1, ou
d) un polynucléotide dont la séquence est une séquence polynucléotidique complémentaire à l'une des séquences polynucléotidiques identifiées sous a), b) ou c) ainsi qu'un excipient pharmaceutiquement acceptable, et étant entendu que :
par "fragment de la séquence du polynucléotide" on entend un polynucléotide comprenant au moins "n" nucléotides consécutifs issus du polynucléotide ID SEQ N°1, où, selon les séquences, n sera égal à 21 nucléotides ou plus, et
par "ayant la même fonction que ladite séquence ID SEQ N°1", on entend une fonction de codage d'un polypeptide antigène de *Mycoplasma pneumoniae.*

## Claims

1. Use of the polypeptide with amino acid sequence ID SEQ No. 2 in order to detect, *in vitro,* in biological samples taken from a human being, the presence of antibodies produced following infection with *Mycoplasma pneumoniae.*

2. Use, in order to detect, *in vitro,* in biological samples taken from a human being, the presence of antibodies produced following infection with *Mycoplasma pneumoniae,* of a polypeptide comprising:
a) a fragment of the amino acid sequence of the polypeptide identified in Claim 1 and having the same function as said sequence ID SEQ No. 2, or
b) an amino acid sequence being at least 60% identical to the amino acid sequence of the polypeptide identified in Claim 1 or with a fragment of the sequence identified under a), and having the same function as said sequence ID SEQ No. 2,
it being assumed that:
"fragment of the amino acid sequence" is understood as meaning a polypeptide comprising at least "n" consecutive amino acids originating from the ID SEQ No. 2 polypeptide where, depending on the sequences, n will be equal to 7 amino acids or more, and
"having the same function as said sequence ID SEQ No. 2" is understood as meaning an antigen function of *Mycoplasma pneumoniae.*

3. The use according to Claim 1 or 2, **characterised in that** the polypeptide is prepared by culturing a host cell comprising a recombinant vecor having inserted into it a polynucleotide encoding for said polypeptide and by isolating said polypeptide from the culture medium.

4. The use according to Claim 3, when it is dependent upon Claim 1, **characterised in that** the polynucleotide is of the sequence ID SEQ No. 1.

5. Use of a polynucleotide with the sequence ID SEQ No. 1 in order to detect, *in vitro,* in biological samples taken from a human being, the presence of antibodies produced following infection with *Mycoplasma pneumoniae.*

6. Use in order to detect, *in vitro,* in biological samples taken from a human being, the presence of antibodies produced following infection with *Mycoplasma pneumoniae,* of a polynucleotide comprising:
a) a fragment of the sequence of the polynucleotide identified in Claim 5 and having the same function as said sequence ID SEQ No. 1, or
b) a polynucleotide sequence being at least 60% identical to the sequence of the polynucleotide identified in Claim 5 or with a fragment of the sequence identified under a), and having the same function as said sequence ID SEQ No. 1, or
c) a polynucleotide sequence complementary to one of the polynucleotide sequences identified in Claim 5 or under points a) or b) above, it being assumed that:
"fragment of the polynucleotide sequence" is understood as meaning a polynucleotide comprising at least "n" consecutive nucleotides originating from the ID SEQ No. 1 polynucleotide, where, depending on the sequences, n will be equal to 21 nucleotides or more, and
"having the same function as said sequence ID SEQ No. 1" is understood as meaning an encoding function of an antigen polypeptide of *Mycoplasma pneumoniae.*

7. A vaccine for the prevention of *Mycoplasma pneumoniae* infections in the human being, comprising as the active principle at least:
a) one polypeptide with the amino acid sequence ID SEQ No. 2, or
b) one polypeptide the sequence of which is a fragment of the amino acid sequence of the polypeptide identified under a) and having the same function as said sequence ID SEQ No. 2, or
c) one polypeptide the sequence of which is an amino acid sequence being at least 60% identical to the amino acid sequence of the polypeptide identified under a) or with the sequence fragment identified under b), and having the same function as said sequence ID SEQ No. 2,
as well as a pharmaceutically acceptable excipient, and
it being assumed that:
"fragment of the amino acid sequence" is understood as meaning a polypeptide comprising at least "n" consecutive amino acids originating from the ID SEQ No. 2 polypeptide where, depending on the sequences, n will be equal to 7 amino acids or more, and
"having the same function as said sequence ID SEQ No. 2" is understood as meaning an antigen function of *Mycoplasma pneumoniae.*

8. A vaccine for the prevention of *Mycoplasma pneumoniae* infections in the human being, comprising as the active principle, at least:
a) one polynucleotide with the sequence ID SEQ No. 1, or
b) one polynucleotide the sequence of which is a fragment of the sequence of the polynucleotide identified under a) and having the same function as said sequence ID SEQ No. 1, or
c) one polynucleotide the sequence of which is a polynucleotide sequence being at least 60% identical to the sequence of the polynucleotide identified under a) or with a sequence fragment identified under b), and having the same function as said sequence ID SEQ No. 1, or
d) one polynucleotide the sequence of which is a polynucleotide sequence complementary to any of the polynucleotide sequences identified under a), b) or c),
as well as a pharmaceutically acceptable excipient, and
it being assumed that:
"fragment of the sequence of the polynucleotide" is understood as meaning a polynucleotide comprising at least "n" consecutive nucleotides originating from the ID SEQ No. 1 polynucleotide where, according to the sequences, n will be equal to 21 nucleotides or more, and
"having the same function as said sequence ID SEQ No. 1" is understood as meaning an encoding function of a *Mycloplasma pneumoniae* antigen polypeptide.

## Patentansprüche

1. Verwendung von Polypeptid der Aminosäurensequenz ID SEQ N°2, um in biologischen Proben, welche von einem Menschen stammen, in vitro das Vorhandensein von Antikörpern zu detektieren, die infolge einer *Mycoplasma* pneumoniae-Infektion erzeugt werden.

2. Verwendung eines Polypeptids in vitro zur Detektion des Vorhandenseins von Antikörpern, die infolge einer *Mycoplasma pneumoniae-*Infektion erzeugt werden, in biologischen Proben, welche von einem Menschen stammen, wobei das Polypeptid umfasst:
a) ein Fragment der Aminosäurensequenz des im Anspruch 1 identifizierten Polypeptids und das dieselbe Funktion wie die Sequenz ID SEQ N°2 hat, oder
b) eine Aminosäurensequenz, die mindestens 60% Identität mit der Aminosäurensequenz des im Anspruch 1 identifizierten Polypeptids oder mit einem unter a) identifizierten Fragment der Sequenz hat und dieselbe Funktion wie die Sequenz ID SEQ N°2 hat,
wobei vereinbart ist, dass:
unter "Fragment der Aminosäurensequenz" ein Polypeptid verstanden wird, das mindestens "n" aufeinanderfolgende, von dem Polypeptid ID SEQ N°2 stammende Aminosäuren umfasst, wobei gemäß den Sequenzen n gleich 7 Aminosäuren oder mehr sein soll und
unter "dieselbe Funktion wie die Sequenz ID SEQ N°2 hat" eine Funktion eines Antigens von *Mycoplasma pneumoniae* verstanden wird.

3. Verwendung nach Anspruch 1 oder 2, **dadurch gekennzeichnet, dass** das Polypeptid durch Kultur einer Wirtszelle, die einen rekombinanten Vektor umfasst, in welchem ein für das Polypeptid kodierendes Polynukleotid insertiert ist, und durch Isolierung des Polypeptids aus dem Kulturmaterial hergestellt wird.

4. Verwendung nach Anspruch 3, wenn er von Anspruch 1 abhängt,
**dadurch gekennzeichnet, dass** das Polynukleotid die Sequenz ID SEQ N°1 aufweist.

5. Verwendung eines Polynukleotids der Sequenz ID SEQ N°1, um in biologischen Proben, welche von einem Menschen stammen, in vitro das Vorhandensein von Antikörpern zu detektieren, die infolge einer *Mycoplasma pneumoniae-Infektion* erzeugt werden.

6. Verwendung eines Polypeptids in vitro zur Detektion des Vorhandenseins von Antikörpern, die infolge einer *Mycoplasma pneumoniae-*Infektion erzeugt werden, in biologischen Proben, welche von einem Menschen stammen, wobei das Polypeptid umfasst:
a) ein Fragment der Sequenz des im Anspruch 5 identifizierten Polynukleotids und das dieselbe Funktion wie die Sequenz ID SEQ N°1 hat, oder
b) eine Polynukleotidsequenz, die mindestens 60% Identität mit der Sequenz des im Anspruch 5 identifizierten Polynukleotids oder mit dem unter a) identifizierten Fragment der Sequenz hat und dieselbe Funktion wie die Sequenz ID SEQ N°1 hat, oder
c) eine Polynukleotidsequenz, welche zu einer im Anspruch 5 oder unter den oben genannten Punkten a) oder b) identifizierten Polynukleotidsequenzen komplementär ist,
wobei vereinbart ist, dass:
unter "Fragment der Sequenz des Polynukleotids" ein Polynukleotid verstanden wird, das mindestens "n" aufeinanderfolgende von dem Polynukleotid ID SEQ N°1 stammende Nukleotide umfasst, wobei gemäß den Sequenzen n gleich 21 Nukleotide oder mehr sein soll, und
unter "dieselbe Funktion wie die Sequenz ID SEQ N°1 hat" eine Kodierfunktion für ein Polypeptid-Antigen von *Mycoplasma pneumoniae* verstanden wird.

7. Impfstoff für die Vorbeugung von *Mycoplasma pneumoniae-*Infektionen bei einem Menschen, als Wirkstoff mindestens umfassend:
a) ein Polypeptid der Aminosäurensequenz ID SEQ N°2 oder
b) ein Polypeptid, dessen Sequenz ein Fragment der Aminosäurensequenz des unter a) identifizierten Polypeptids ist und das dieselbe Funktion wie die Sequenz ID SEQ N°2 hat, oder
c) ein Polypeptid, dessen Sequenz eine Aminosäurensequenz ist, die mindestens 60% Identität mit der Aminosäurensequenz des unter a) identifizierten Polypeptids hat oder mit dem unter b) identifizierten Sequenzfragment hat und das dieselbe Funktion wie die Sequenz ID SEQ N°2 hat,
sowie einen annehmbaren pharmazeutischen Grundstoff, und wobei verabredet ist, dass:
unter "Fragment der Aminosäurensequenz" ein Polypeptid verstanden wird, das mindestens "n" aufeinanderfolgende, von dem Polypeptid ID SEQ N°2 stammende Aminosäuren umfasst, wobei gemäß den Sequenzen n gleich 7 Aminosäuren oder mehr sein soll und
unter "dieselbe Funktion wie die Sequenz ID SEQ N°2 hat" eine Funktion des Antigens der *Mycoplasma pneumoniae* verstanden wird.

8. Impfstoff für die Vorbeugung von *Mycoplasma pneumoniae-*Infektionen bei einem Menschen, als Wirkstoff mindestens umfassend:
a) ein Polynukleotid der Sequenz ID SEQ N°1 oder
b) ein Polynukleotid, dessen Sequenz ein Fragment der Sequenz des unter a) identifizierten Polynukleotids ist und das dieselbe Funktion wie die Sequenz ID SEQ N°1 hat, oder
c) ein Polynukleotid, dessen Sequenz eine Polynukleotidsequenz ist, die mindestens 60% Identität mit der Sequenz des unter a) identifizierten Polynukleotids oder mit dem unter b) identifizierten Sequenzfragment hat und das dieselbe Funktion wie die Sequenz ID SEQ N°1 hat, oder
d) ein Polynukleotid, dessen Sequenz eine zu einer der unter a), b) oder c) identifizierten Polynukleotidsequenzen komplementäre Polynukleotidsequenz ist,
sowie einen annehmbaren pharmazeutischen Grundstoff, und
wobei verabredet ist, dass:
unter "Fragment der Sequenz des Polynukleotids" ein Polynukleotid verstanden wird, das mindestens "n" aufeinanderfolgende, von dem Polynukleotid ID SEQ N°1 stammende Nukleotide umfasst, wobei gemäß den Sequenzen n gleich 21 Nukleotide oder mehr sein soll, und
unter "dieselbe Funktion wie die Sequenz ID SEQ N°1 hat" eine Kodierfunktion für ein Polypeptid-Antigen von *Mycoplasma pneumoniae* verstanden wird.


### LISTAGE DE SEQUENCES

<110> ABAG
   Université Victor Segalen Bordeaux 2
<120> Utilisation de la protéine 7F4 dans le diagnostic d'infections à Mycoplasma pneumoniae
<130> B4325
<150> FR0608734
   <151> 2006-10-05
<160> 2
<170> PatentIn version 3.3
<210> 1
   <211> 1422
   <212> DNA
   <213> Mycoplasma pneumoniae
<220>
   <221> CDS
   <222> (1)..(1422)
<400> 1
   aaa aag gaa aac att aca tac ggt aag gtc cac caa gtg att ggc ccg      48
   Lys Lys Glu Asn Ile Thr Tyr Gly Lys Val His Gln Val Ile Gly Pro
   1               5                   10                  15
   gta gtt gat gtc atc ttt acg gaa agt agt cag tta ccc cgc att tac      96
   Val Val Asp Val Ile Phe Thr Glu Ser Ser Gln Leu Pro Arg Ile Tyr
               20                  25                  30
   gat tgt ttg agt gtt aag tta gct ggg gaa gaa ctg ttt ttg gaa gcc     144
   Asp Cys Leu Ser Val Lys Leu Ala Gly Glu Glu Leu Phe Leu Glu Ala
           35                  40                  45
   gcg cag cta ata ggc gat gac att gtg cgc tgc att gct ttg ggt cca     192
   Ala Gln Leu Ile Gly Asp Asp Ile Val Arg Cys Ile Ala Leu Gly Pro
       50                  55                  60
   act gaa ggg tta gca cgc aac gaa aag gtg act aac tat aac cac ccg     240
   Thr Glu Gly Leu Ala Arg Asn Glu Lys Val Thr Asn Tyr Asn His Pro
   65                  70                  75                  80
   att gaa gtc cca gtt ggc aaa aac gtc ttg ggc cgg atg ttt aat gtt     288
   Ile Glu Val Pro Val Gly Lys Asn Val Leu Gly Arg Met Phe Asn Val
                   85                  90                  95
   ttg ggt aaa ccc att gac ggg aag gag gag tta ccc aaa aaa cca caa     336
   Leu Gly Lys Pro Ile Asp Gly Lys Glu Glu Leu Pro Lys Lys Pro Gln
               100                 105                 110
   ctc ccg att cac cgc aaa cca cct tcg ttt gat gac cag tcc aat acg     384
   Leu Pro Ile His Arg Lys Pro Pro Ser Phe Asp Asp Gln Ser Asn Thr
           115                 120                 125
   cta gaa atc ttt gaa aca ggg att aag gtg att gac ttg ctc act ccc      432
   Leu Glu Ile Phe Glu Thr Gly Ile Lys Val Ile Asp Leu Leu Thr Pro
       130                 135                 140
   tat gcc cgt ggt ggt aag att ggt tta ttt ggt ggt gct ggt gtt ggt      480
   Tyr Ala Arg Gly Gly Lys Ile Gly Leu Phe Gly Gly Ala Gly Val Gly
   145                 150                 155                 160
   aaa acg gtt tta gtg caa gag tta atc cac aac att gct aag gaa cac      528
   Lys Thr Val Leu Val Gln Glu Leu Ile His Asn Ile Ala Lys Glu His
                   165                 170                 175
   tct ggt tta agt gtg ttt gct ggt gtg ggg gaa cgc acc cgg gaa ggt      576
   Ser Gly Leu Ser Val Phe Ala Gly Val Gly Glu Arg Thr Arg Glu Gly
               180                 185                 190
   aat gat ctc tac tat gaa atg atc cag ggt ggg gtg att gac aaa act      624
   Asn Asp Leu Tyr Tyr Glu Met Ile Gln Gly Gly Val Ile Asp Lys Thr
           195                 200                 205
   gct tta gtg ttt ggt cag atg aac gaa ccc cca gga gca cgg atg cgg      672
   Ala Leu Val Phe Gly Gln Met Asn Glu Pro Pro Gly Ala Arg Met Arg
       210                 215                 220
   gtc gct tta acg gcg ctt act atg gcc gaa tac ttc cgt gac cat gac      720
   Val Ala Leu Thr Ala Leu Thr Met Ala Glu Tyr Phe Arg Asp His Asp
   225                 230                 235                 240
   aac cag gat gtc ttg ctc ttc att gac aac atc ttc cgt ttt acc caa      768
   Asn Gln Asp Val Leu Leu Phe Ile Asp Asn Ile Phe Arg Phe Thr Gln
                   245                 250                 255
   gct ggt agt gag gta tca gca ctg tta ggt cgg atg cca tca gcc gtg      816
   Ala Gly Ser Glu Val Ser Ala Leu Leu Gly Arg Met Pro Ser Ala Val
               260                 265                 270
   ggt tac caa cca acg ttg gcg act gaa atg ggg caa tta caa gag cgg      864
   Gly Tyr Gln Pro Thr Leu Ala Thr Glu Met Gly Gln Leu Gln Glu Arg
           275                 280                 285
   att gct tcc act aaa acc ggt tcg att acc tcg gtg caa gcg atc tat      912
   Ile Ala Ser Thr Lys Thr Gly Ser Ile Thr Ser Val Gln Ala Ile Tyr
       290                 295                 300
   gtg cca gct gat gac ttg act gac ccc gct ccg gct acc acc ttt acc      960
   Val Pro Ala Asp Asp Leu Thr Asp Pro Ala Pro Ala Thr Thr Phe Thr
   305                 310                 315                 320
   cac ctg gat gct aaa acg gtg tta gac cgg aac ata gca gcg ttg gga     1008
   His Leu Asp Ala Lys Thr Val Leu Asp Arg Asn Ile Ala Ala Leu Gly
                   325                 330                 335
   att ttt cca gcg atc aat ccc tta gag tct acc agt cgc tta ttg gat     1056
   Ile Phe Pro Ala Ile Asn Pro Leu Glu Ser Thr Ser Arg Leu Leu Asp
               340                 345                 350
   cct aat att gtc ggg att aac cac tat aag gta gcg tta ggg gtg caa     1104
   Pro Asn Ile Val Gly Ile Asn His Tyr Lys Val Ala Leu Gly Val Gln
           355                 360                 365
   aac atc tta cag cgc ttt gcg gaa cta caa gac att att gcc atc ttg      1152
   Asn Ile Leu Gln Arg Phe Ala Glu Leu Gln Asp Ile Ile Ala Ile Leu
       370                 375                 380
   gga att gat gag tta gcg gat gag gac aag atc att gtg gaa cgg gca      1200
   Gly Ile Asp Glu Leu Ala Asp Glu Asp Lys Ile Ile Val Glu Arg Ala
   385                 390                 395                 400
   cgc cgg atc cgc aac ttc ctc tcc caa ccg ttc ttt gta gcg gaa aag      1248
   Arg Arg Ile Arg Asn Phe Leu Ser Gln Pro Phe Phe Val Ala Glu Lys
                   405                 410                 415
   ttc tcg ggg att gct ggg aag tat gtc cct tta agt gac acg atc caa      1296
   Phe Ser Gly Ile Ala Gly Lys Tyr Val Pro Leu Ser Asp Thr Ile Gln
               420                 425                 430
   tcg ttt aag gaa atc ttg gac ggt aag cat gat gat ctc ccc gaa cag      1344
   Ser Phe Lys Glu Ile Leu Asp Gly Lys His Asp Asp Leu Pro Glu Gln
           435                 440                 445
   gcc ttc ttc ttt gtt ggt acc att cag gaa gca gtg gag aaa gcg aag      1392
   Ala Phe Phe Phe Val Gly Thr Ile Gln Glu Ala Val Glu Lys Ala Lys
       450                 455                 460
   cgg ctt aaa aaa gct act gtt gag gag aaa                              1422
   Arg Leu Lys Lys Ala Thr Val Glu Glu Lys
   465                 470
<210> 2
   <211> 474
   <212> PRT
   <213> Mycoplasma pneumoniae
<400> 2
   Lys Lys Glu Asn Ile Thr Tyr Gly Lys Val His Gln Val Ile Gly Pro
   1               5                   10                  15
   Val Val Asp Val Ile Phe Thr Glu Ser Ser Gln Leu Pro Arg Ile Tyr
               20                  25                  30
   Asp Cys Leu Ser Val Lys Leu Ala Gly Glu Glu Leu Phe Leu Glu Ala
           35                  40                  45
   Ala Gln Leu Ile Gly Asp Asp Ile Val Arg Cys Ile Ala Leu Gly Pro
       50                  55                  60
   Thr Glu Gly Leu Ala Arg Asn Glu Lys Val Thr Asn Tyr Asn His Pro
   65                  70                  75                  80
   Ile Glu Val Pro Val Gly Lys Asn Val Leu Gly Arg Met Phe Asn Val
                   85                  90                  95
   Leu Gly Lys Pro Ile Asp Gly Lys Glu Glu Leu Pro Lys Lys Pro Gln
               100                 105                 110
   Leu Pro Ile His Arg Lys Pro Pro Ser Phe Asp Asp Gln Ser Asn Thr
           115                 120                 125
   Leu Glu Ile Phe Glu Thr Gly Ile Lys Val Ile Asp Leu Leu Thr Pro
       130                 135                 140
   Tyr Ala Arg Gly Gly Lys Ile Gly Leu Phe Gly Gly Ala Gly Val Gly
   145                 150                 155                 160
   Lys Thr Val Leu Val Gln Glu Leu Ile His Asn Ile Ala Lys Glu His
                   165                 170                 175
   Ser Gly Leu Ser Val Phe Ala Gly Val Gly Glu Arg Thr Arg Glu Gly
               180                 185                 190
   Asn Asp Leu Tyr Tyr Glu Met Ile Gln Gly Gly Val Ile Asp Lys Thr
           195                 200                 205
   Ala Leu Val Phe Gly Gln Met Asn Glu Pro Pro Gly Ala Arg Met Arg
       210                 215                 220
   Val Ala Leu Thr Ala Leu Thr Met Ala Glu Tyr Phe Arg Asp His Asp
   225                 230                 235                 240
   Asn Gln Asp Val Leu Leu Phe Ile Asp Asn Ile Phe Arg Phe Thr Gln
                   245                 250                 255
   Ala Gly Ser Glu Val Ser Ala Leu Leu Gly Arg Met Pro Ser Ala Val
               260                 265                 270
   Gly Tyr Gln Pro Thr Leu Ala Thr Glu Met Gly Gln Leu Gln Glu Arg
           275                 280                 285
   Ile Ala Ser Thr Lys Thr Gly Ser Ile Thr Ser Val Gln Ala Ile Tyr
       290                 295                 300
   Val Pro Ala Asp Asp Leu Thr Asp Pro Ala Pro Ala Thr Thr Phe Thr
   305                 310                 315                 320
   His Leu Asp Ala Lys Thr Val Leu Asp Arg Asn Ile Ala Ala Leu Gly
                   325                 330                 335
   Ile Phe Pro Ala Ile Asn Pro Leu Glu Ser Thr Ser Arg Leu Leu Asp
               340                 345                 350
   Pro Asn Ile Val Gly Ile Asn His Tyr Lys Val Ala Leu Gly Val Gln
           355                 360                 365
   Asn Ile Leu Gln Arg Phe Ala Glu Leu Gln Asp Ile Ile Ala Ile Leu
       370                 375                 380
   Gly Ile Asp Glu Leu Ala Asp Glu Asp Lys Ile Ile Val Glu Arg Ala
   385                 390                 395                 400
   Arg Arg Ile Arg Asn Phe Leu Ser Gln Pro Phe Phe Val Ala Glu Lys
                   405                 410                 415
   Phe Ser Gly Ile Ala Gly Lys Tyr Val Pro Leu Ser Asp Thr Ile Gln
               420                 425                 430
   Ser Phe Lys Glu Ile Leu Asp Gly Lys His Asp Asp Leu Pro Glu Gln
           435                 440                 445
   Ala Phe Phe Phe Val Gly Thr Ile Gln Glu Ala Val Glu Lys Ala Lys
       450                 455                 460
   Arg Leu Lys Lys Ala Thr Val Glu Glu Lys
   465                 470